# EUROPÄISCHE PATENTSCHRIFT

(11) **EP 2 262 430 B1**
(45) Veröffentlichungstag und Bekanntmachung des Hinweises auf die Patenterteilung: **28.12.2016**
(21) Anmeldenummer: 09732430.5
(22) Anmeldetag: 31.03.2009
(51) Int. Cl.: A61B 90/90, A61B 90/92, A61B 90/94, A61F 9/013

(54) **MEDIZINISCHES HANDGERÄT**
HAND-HELD MEDICAL APPLIANCE
APPAREIL MANUEL MÉDICAL

(30) Priorität: 16.04.2008 DE 102008019312
(43) Veröffentlichungstag der Anmeldung: 22.12.2010
(73) Patentinhaber: Geuder AG, 69126 Heidelberg (DE)
(72) Erfinder: GEUDER, Volker, 69126 Heidelberg (DE); FRAUENFELD, Dieter, 69121 Heidelberg (DE); DRAHEIM, René, 69207 Sandhausen (DE)
(74) Vertreter: Patent- und Rechtsanwälte Ullrich & Naumann
(86) Internationale Anmeldenummer: PCT/DE2009/000406
(87) Internationale Veröffentlichungsnummer: WO 2009/127185

(56) Entgegenhaltungen:
- EP-A- 0 054 653
- EP-A- 1 393 685
- WO-A-2007/005313
- US-A- 3 747 603
- US-A- 5 690 658
- US-A1- 2003 109 858

## Beschreibung

Die Erfindung betrifft ein medizinisches Handgerät zur Anwendung in der Ophthalmologie, mit einem Griff zum Halten des Geräts und einem vom Griff getragenen Werkzeug.

Medizinische Handgeräte sind in den unterschiedlichsten Ausprägungen aus der Praxis bekannt. Solche Handgeräte sind - je nach Bedarf - mit unterschiedlichen Werkzeugen ausgestattet, wobei häufig das Werkzeug austauschbar ist. Bei den Werkzeugen kann es sich um Scheren, Messer bzw. Skalpelle, Beleuchtungseinrichtungen, Absaugeinrichtungen, etc. handeln. Lediglich beispielhaft sei auf die EP 0 870 472 A1 verwiesen, aus der ein Diamant-Skalpell bekannt ist.

Ein ophthalmologisches Handgerät, welches die Merkmale des Oberbegriffs des Anspruchs 1 beinhaltet, ist aus WO 2007/005313 bekannt.

Bei den bekannten Handgeräten der gattungsbildenden Art ist häufig die Griffoberfläche angerauht oder mit einer Riffelung versehen, um nämlich die Griffigkeit des Handgeräts zu verbessern. Da nicht selten die gesamte Griffoberfläche mit der Riffelung versehen oder angeraut ist, ist die Reinigung eines solchen Handgeräts erschwert. Außerdem bietet ein solches Handgerät der das Gerät handhabenden Person keinerlei Orientierungshilfe zum Ergreifen des Geräts. Auch fehlt diesen Geräten ein das Gerät individualisierendes bzw. identifizierendes Merkmal.

Der vorliegenden Erfindung liegt daher die Aufgabe zugrunde, das gattungsbildende medizinische Handgerät derart auszugestalten und weiterzubilden, dass bei hinreichend guter Griffigkeit die Reinigung des Geräts gut möglich und gleichzeitig eine Identifizierung betreffend das Handgerät selbst oder das vom Handgerät getragene Werkzeug möglich ist.

Voranstehende Aufgabe ist durch Merkmale des Patentanspruchs 1 gelöst. Danach ist das gattungsbildende medizinische Handgerät dadurch gekennzeichnet, dass die Haltebereiche eine das Werkzeug identifizierende Kodierung umfassen und dass die Kodierung durch die Farbe der Haltebereiche definiert ist.

Erfindungsgemäß ist erkannt worden, dass die Griffigkeit bereits dann hinreichend gut ist, wenn die Griffigkeit begünstigende Haltebereiche im bzw. am Griff ausgebildet sind. Eine die komplette Oberfläche betreffende Riffelung ist nicht erforderlich.

Des Weiteren ist erkannt worden, dass es möglich ist, den Haltebereichen eine weitere Funktion zuzuordnen, nämlich eine das Werkzeug identifizierende Kodierung, die durch die Haltebereich selbst gebildet bzw. definiert ist. Somit dienen die Haltebereiche einerseits zur Begünstigung der Griffigkeit und andererseits zur visuellen Erkennbarkeit des jeweiligen Handgeräts bzw. des darin befindlichen Werkzeugs.

Das Werkzeug kann beispielsweise an einem einteiligen Handgriff unmittelbar befestigt sein. Ebenso ist es denkbar, dass sich an den Griff ein das Werkzeug tragender, auswechselbarer Kopf anschließt, der mit entsprechenden Haltebereichen ausgestattet ist. Zum Werkzeugwechsel wird der gesamte Kopf getauscht, der einerseits die Griffigkeit begünstigende Haltebereiche und gleichzeitig - mit den Haltebereichen - eine das Werkzeug identifizierende Kodierung umfasst. Die Haltebereiche können beispielsweise eine Handhabung des Kopfes begünstigen, und zwar nicht nur beim Anbringen oder Entfernen des Kopfes. Beim Einsatz könnte beispielsweise die Notwendigkeit entstehen, den Kopf definiert zu drehen. Auch insoweit sind dort entsprechende Haltebereiche erforderlich bzw. von Vorteil.

Die Haltebereiche sind in weiter vorteilhafter Weise zonal ausgebildet, wobei diese im Konkreten symmetrisch entlang dem Umfang ausgebildet und angeordnet sein können. Auch ist es denkbar, eine asymmetrische Anordnung vorzusehen, um nämlich der handhabenden Person vorzugeben, in welcher Winkelstellung das Handgerät zu halten hat. Beliebige symmetrische und asymmetrische Ausgestaltungen und Anordnungen der Haltebereiche sind denkbar.

Die Haltebereiche können weiter haptisch erfassbare Informationen beinhalten. Dazu ist es von Vorteil, wenn sich die Haltebereiche gegenüber der sonstigen Griffoberfläche abheben. Im Konkreten ist es denkbar, dass die Haltebereiche gegenüber der sonstigen Griffoberfläche erhaben sind. Dabei können sie punktuell erhaben bzw. genoppt sein. Rasterartige Ausführungen sind denkbar. Auch könnte mit den Noppen eine Art Blindenschrift realisiert sein, um nämlich eine eindeutige Identifizierung vornehmen zu können.

Alternativ ist es denkbar, dass die Haltebereiche gegenüber der sonstigen Griffoberfläche vertieft sind. Auch dadurch lässt sich eine Kodierung realisieren. Im Konkreten könnten die Haltebereiche als Mulden zum Einlegen jeweils eines Fingers ausgeführt sein. Auch dadurch kann das Ergreifen des Handgeräts vorgegeben sein.

Im Rahmen einer weiteren Alternative ist es denkbar, dass die Haltebereiche in der Ebene der Griffoberfläche liegen. Sie könnten sich durch Rauhigkeit bzw. Oberflächenbeschaffenheit gegenüber der sonstigen Griffoberfläche abheben.

Grundsätzlich ist es denkbar, dass die Haltebereiche ergonomisch ausgeformt und entsprechend angeordnet sind.

Wie bereits zuvor ausgeführt, ist es von Vorteil, wenn die Kodierung zusätzlich durch die Haptik der Haltbereiche definiert ist. Dazu ist es von weiterem Vorteil, wenn die Kodierung durch die Form und/oder Anordnung der Halterbeiche definiert ist. Die Kodierung ist durch die Farbe der Haltebereiche definiert, wobei dies durch unterschiedliche Farben bzw. durch eine unterschiedliche Farbfolge in den jeweiligen Haltebereichen möglich ist.

Die Haltebereiche sind aus einem rutschhemmenden bzw. rutschfesten Material hergestellt. Dabei ist es von Vorteil, wenn diese aus weichem Kunststoff, weicher als das Material des Griffs, oder aus Gummi bestehen. Sofern es sich bei dem Griff und/oder bei dem Kopf um Kunststoffteile handelt, ist es in Bezug auf die Fertigung von Vorteil, wenn die Haltebereiche im Overmouldingverfahren, d. h. spritzgusstechnisch, hergestellt sind.

Es gibt nun verschiedene Möglichkeiten, die Lehre der vorliegenden Erfindung in vorteilhafter Weise auszugestalten und weiterzubilden. Dazu ist einerseits auf die nachgeordneten Patentansprüche und andererseits auf die nachfolgende Erläuterung zweier Ausführungsbeispiele des erfindungsgemäßen medizinischen Handgeräts anhand der Zeichnung zu verweisen. In Verbindung mit der Erläuterung der bevorzugten Ausführungsbeispiele des Handgeräts werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel eines chirurgischen Handgeräts zur Anwendung in der Ophthalmologie, wobei das Handgerät einen Griff 1 zum Halten des Geräts und ein vom Griff 1 getragenes Werkzeug 2 umfasst,
- Fig. 2: in einer schematischen Ansicht den austauschbaren Kopf eines chirurgischen Handgeräts, ausgestattet mit Haltebereichen nebst Kodierung.

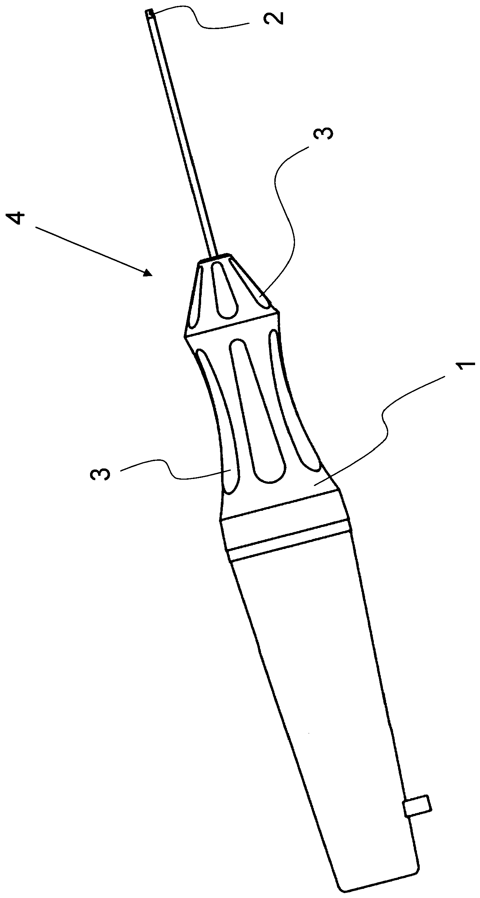

Fig. 1 zeigt deutlich, dass der Griff 1 die Griffigkeit begünstigende Haltebereiche 3 umfasst, wobei die Haltebereiche 3 eine das Werkzeug 2 identifizierende Kodierung umfassen. Die Kodierung ergibt sich aus der Haptik einerseits und der Farbe der Haltebereiche 3 andererseits.

Bei dem in Fig. 1 gezeigten Ausführungsbeispiel schließt sich an den Griff 1 ein das Werkzeug 2 tragender auswechselbarer Kopf 4 an. Auch der Kopf 4 ist mit Haltebereichen 3 ausgestattet, um nämlich das Werkzeug 2 gemeinsam mit dem Kopf 4 besser fassen und drehen zu können.

Fig. 1 zeigt des Weiteren deutlich, dass die Haltebereiche 3 symmetrisch entlang dem Umfang des Griffs 1 und des Kopfes 4 angeordnet bzw. ausgebildet sind. Die Haltebereiche 3 können erhaben, vertieft oder exakt in der Griffoberfläche liegend ausgeführt sein.

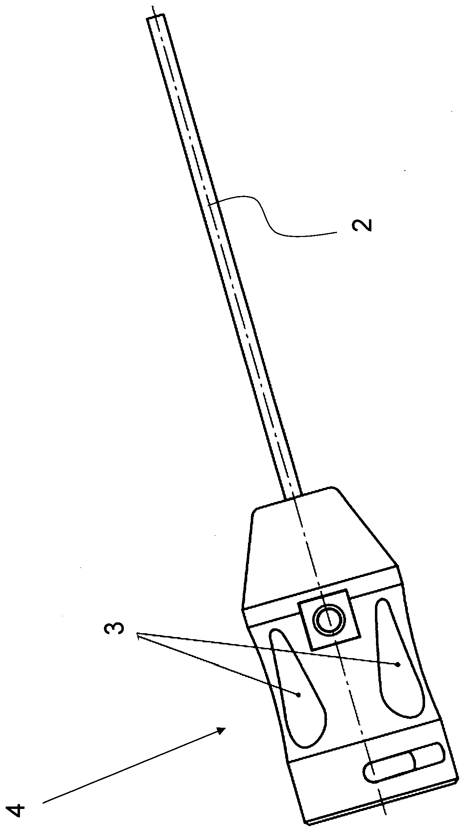

Fig. 2 zeigt ein weiteres Ausführungsbeispiel eines medizinischen Handgeräts, genauer gesagt, den das Werkzeug 2 tragenden Kopf 4, der mit die Griffigkeit begünstigenden Haltebereichen 3 ausgestattet ist. Die Haptik und die Farbgebung der Haltebereiche 3 dienen zur Kodierung bzw. ermöglichen der handhabenden Person ein eindeutiges Erkennen des Kopfes bzw. des vom Kopf 4 gehaltenen Werkzeugs 2.

Hinsichtlich weiterer Merkmale, die sich den Figuren nicht entnehmen lassen, sei zur Vermeidung von Wiederholungen auf den allgemeinen Teil der Beschreibung verwiesen.

Schließlich sei angemerkt, dass die voranstehend erörterten Ausführungsbeispiele lediglich der beispielhaften Erläuterung des erfindungsgemäßen Handgeräts dienen, diese jedoch nicht auf die Ausführungsbeispiele einschränken.

### Bezugszeichenliste

- 1: Griff
- 2: Werkzeug
- 3: Haltebereich
- 4: Kopf

## Patentansprüche

1. Ophthalmologisches Handgerät mit einem Griff (1) zum Halten des Geräts und einem vom Griff (1) getragenen Werkzeug (2), wobei der Griff (1) die Griffigkeit begünstigende Haltebereiche (3) aufweist und wobei die Haltebereiche (3) aus einem rutschhemmenden Material hergestellt sind,
**dadurch gekennzeichnet, dass** die Haltebereiche (3) eine das Werkzeug (2) identifizierende Kodierung umfassen und dass die Kodierung durch die Farbe der Haltebereiche (3) definiert ist.

2. Handgerät nach Anspruch 1, **dadurch gekennzeichnet, dass** sich an den Griff (1) ein das Werkzeug (2) tragender, auswechselbarer Kopf (4) anschließt, der mit Haltebereichen (4) ausgestattet ist.

3. Handgerät nach Anspruch 1 oder 2, **dadurch gekennzeichnet, dass** die Haltebereiche (3) zonal ausgebildet sind.

4. Handgerät nach einem der Ansprüche 1 bis 3, **dadurch gekennzeichnet, dass** die Haltebereiche (3) symmetrisch entlang dem Umfang ausgebildet und angeordnet sind.

5. Handgerät nach einem der Ansprüche 1 bis 4, **dadurch gekennzeichnet, dass** die Haltebereiche (3) gegenüber der sonstigen Griffoberfläche erhaben sind, wobei die Haltebereiche (3) gegenüber der sonstigen Griffoberfläche punktuell erhaben bzw. genoppt sein können.

6. Handgerät nach einem der Ansprüche 1 bis 4, **dadurch gekennzeichnet, dass** die Haltebereiche (3) gegenüber der sonstigen Griffoberfläche vertieft sind, wobei die Haltebereiche (3) als Mulden zum Einlegen jeweils eines Fingers ausgeführt sein können.

7. Handgerät nach einem der Ansprüche 1 bis 4, **dadurch gekennzeichnet, dass** die Haltebereiche (3) in der Ebene der Griffoberfläche liegen.

8. Handgerät nach einem der Ansprüche 1 bis 7, **dadurch gekennzeichnet, dass** die Haltebereiche (3) ergonomisch ausgeformt und angeordnet sind.

9. Handgerät nach einem der Ansprüche 1 bis 8, **dadurch gekennzeichnet, dass** die Kodierung durch die Haptik der Haltebereiche (3) definiert ist.

10. Handgerät nach Anspruch 9, **dadurch gekennzeichnet, dass** die Kodierung durch die Form und Anordnung der Haltebereiche definiert ist.

11. Handgerät nach einem der Ansprüche 1 bis 11, **dadurch gekennzeichnet, dass** die Kodierung durch unterschiedliche Farben bzw. durch eine unterschiedliche Farbfolge definiert ist.

12. Handgerät nach Anspruch 1, **dadurch gekennzeichnet, dass** die Haltebereiche (3) aus weichem Kunststoff oder aus Gummi bestehen.

13. Handgerät nach Anspruch 1 oder 11, **dadurch gekennzeichnet, dass** der Griff (1) und/oder der Kopf (4) aus Kunststoff gefertigt sind und dass die Haltebereiche (3) im Overmouldingverfahren, d.h. spritzgusstechnisch, erzeugt sind.

## Claims

1. Handheld ophthalmological device comprising a handle (1) for holding the device and a tool (2) borne by the handle (1), the handle (1) comprising holding regions (3) that promote grip, and the holding regions (3) being made from non-slip material, **characterised in that** the holding regions (3) comprise a coding that identifies the tool (2), and **in that** the coding is defined by the colour of the holding regions (3).

2. Handheld device according to claim 1, **characterised in that** a replaceable head (4) bearing the tool (2) is attached to the handle (1) and provided with holding regions (4).

3. Handheld device according to either claim 1 or claim 2, **characterised in that** the holding regions (3) are formed in the manner of zones.

4. Handheld device according to any one of claims 1 to 3, **characterised in that** the holding regions (3) are formed and arranged symmetrically over the periphery.

5. Handheld device according to any one of claims 1 to 4, **characterised in that** the holding regions (3) are raised in relation to the remainder of the handle surface, it being possible for the holding regions (3) to be raised or nubby in relation to the remainder of the handle surface at various points.

6. Handheld device according to any one of claims 1 to 4, **characterised in that** the holding regions (3) are recessed in relation to the remainder of the handle surface, it being possible for the holding regions (3) to be formed as depressions into each of which one finger, respectively, is placed.

7. Handheld device according to any one of claims 1 to 4, **characterised in that** the holding regions (3) are in the plane of the handle surface.

8. Handheld device according to any one of claims 1 to 7, **characterised in that** the holding regions (3) are formed and arranged in an ergonomic manner.

9. Handheld device according to any one of claims 1 to 8, **characterised in that** the coding is defined by the haptics of the holding regions (3).

10. Handheld device according to claim 9, **characterised in that** the coding is defined by the shape and arrangement of the holding regions.

11. Handheld device according to any one of claims 1 to 11, **characterised in that** the coding is defined by different colours or by a different colour sequence.

12. Handheld device according to claim 1, **characterised in that** the holding regions (3) are made of soft plastics material or of rubber.

13. Handheld device according to either claim 1 or claim 11, **characterised in that** the handle (1) and/or the head (4) are made from plastics material, and **in that** the holding regions (3) are produced in an overmoulding process, i.e. by injection moulding.

## Revendications

1. Appareil ophtalmologique portatif avec une poignée (1) pour tenir l'appareil et un outil (2) supporté par la poignée (1), la poignée (1) comprenant des zones de maintien (3) favorisant la préhension et les zones de maintien (3) étant constituées d'un matériau anti-dérapant,
**caractérisé en ce que** les zones de maintien (3) comprennent un codage identifiant l'outil (2) et **en ce que** le codage est défini par la couleur des zones de maintien (3).

2. Appareil portatif selon la revendication 1, **caractérisé en ce qu'**une tête interchangeable (4) supportant l'outil (2), qui est équipée de zones de maintien (4), se raccorde à la poignée (1).

3. Appareil portatif selon la revendication 1 ou 2, **caractérisé en ce que** les zones de maintien (3) sont réalisées par zones.

4. Appareil portatif selon l'une des revendications 1 à 3, **caractérisé en ce que** les zones de maintien (3) sont réalisées et disposées symétriquement le long de la circonférence.

5. Appareil portatif selon l'une des revendications 1 à 4, **caractérisé en ce que** les zones de maintien (3) sont surélevées par rapport au reste de la surface de la poignée, les zones de maintien (3) pouvant être surélevées ponctuellement par rapport au reste de la surface de la poignée ou pourvues de picots.

6. Appareil portatif selon l'une des revendications 1 à 4, **caractérisé en ce que** les zones de maintien (3) sont abaissées par rapport au reste de la surface de la poignée, les zones de maintien (3) pouvant être réalisées comme des dépressions pour l'insertion d'un doigt.

7. Appareil portatif selon l'une des revendications 1 à 4, **caractérisé en ce que** les zones de maintien (3) se trouvent dans le plan de la surface de la poignée.

8. Appareil portatif selon l'une des revendications 1 à 7, **caractérisé en ce que** les zones de maintien (3) sont formées et disposées de manière ergonomique.

9. Appareil portatif selon l'une des revendications 1 à 8, **caractérisé en ce que** le codage est défini par le toucher des zones de maintien (3).

10. Appareil portatif selon la revendication 9, **caractérisé en ce que** le codage est défini par la forme et la disposition des zones de maintien.

11. Appareil portatif selon l'une des revendications 1 à 11, **caractérisé en ce que** le codage est défini par des couleurs différentes ou par une suite de différentes couleurs.

12. Appareil portatif selon la revendication 1, **caractérisé en ce que** les zones de maintien (3) sont constituées d'une matière plastique ou d'un caoutchouc souple.

13. Appareil portatif selon l'une des revendications 1 à 11, **caractérisé en ce que** la poignée (1) et/ou la tête (4) sont constituées de matière plastique et **en ce que** les zones de maintien (3) sont réalisées à l'aide d'un procédé de surmoulage, c'est-à-dire avec une technique de moulage par injection.
